# EUROPEAN PATENT SPECIFICATION

(11) **EP 1 448 753 B1**
(45) Date of publication and mention of the grant of the patent: **21.06.2006**
(21) Application number: 02787746.3
(22) Date of filing: 20.11.2002
(51) Int. Cl.: C11D 1/722, C11D 3/37

(54) **CONDITIONING SOLUTION FOR CONTACT LENSES**
KONDITIONIERUNGSLÖSUNG FÜR KONTAKTLINSEN
SOLUTION DE CONDITIONNEMENT POUR LENTILLES DE CONTACT

(30) Priority: 21.11.2001 US 332065 P
(43) Date of publication of application: 25.08.2004
(73) Proprietor: Novartis AG, 4056 Basel (CH); Novartis Pharma GmbH, 1235 Vienna (AT)
(72) Inventor: TSAO, Fu-Pao, Lawrenceville, GA 30043 (US)
(74) Representative: de Weerd, Petrus G.W.
(86) International application number: PCT/EP2002/013017
(87) International publication number: WO 2003/043668

(56) References cited:
- WO-A-02/26922
- WO-A-02/055118
- US-A- 5 411 597
- US-A- 5 773 396

## Description

### BACKGROUND OF THE INVENTION

The present invention relates to an improved contact lens conditioning solution. More particularly, it relates to a solution that renders the contact lens surface more wettable so that proteins, lipids, and other tear film substituents do not adhere and form deposits on the lens surface.

Contact lenses are typically made of plastic and, therefore, are hydrophobic or water repellant. Since the use of the first contact lenses, there has been a recognized need to use conditioning agents for contact lenses to render the contact lenses more hydrophilic or "wettable." The purpose of these conditioning agents is to render the lens surface more wettable so that proteins, lipids, and other tear film substituents do not adhere and form deposits thereon. Such deposits reduce the comfort and safety of the lens, and also interfere with optical clarity since it is important that the tear fluid spread evenly over the surface of the lens.

Surface-active agents have been employed in conditioning solutions in an attempt to alleviate the problems associated with contact lens wear, such as insertion comfort and dry eye. Surface-active agents are adsorbed on the lens surface and allow ready spreading of tears when the lenses are inserted, thus making them more comfortable to wear. Representative wetting agents and viscosity modifiers have included: cellulose derivatives, such as cationic cellulosic polymers, hydroxypropyl methylcellulose, hydroxyethylcellulose and methylcellulose; polyols, such as polyethylene glycol, glycerine and polyethylene oxide (PEO) containing polymers; polyvinyl alcohol; and polyvinyl pyrrolidone. Such additives may be used in a wide range of concentrations as is known in the art. These types of agents, however, do not adsorb to a significant level to the lens, and therefore do not provide prolonged comfort.

Polymers, which provide a more prolonged comfort level typically need to be employed in high concentration to create a higher viscosity, and thereby prolong the retention of the polymer. The use of these high viscosity agents, however, may cause blurring of vision when the lens is first placed in the eye, and also creating a sticky feeling of the lens to the user, making lens insertion and handling difficult. More hydrophobic polymers can adsorb more readily to the lens, and can be formulated at lower concentrations to provide better lubrication. The disadvantage, however, of a more hydrophobic polymer is that the polymer may also act as a substrate for deposits and as a consequence, make the lens more prone to filming and lipid deposit.

Therefore, there exists a need for a conditioning solution that renders a contact lens surface more wettable so that proteins, lipids, and other tear film substituents do not adhere thereto and form deposits. There is a further need for such a solution that may be used on hard, RGP and hydrogel (soft) contact lenses. Such a conditioning solution would greatly enhance the cleaning operations now required.

Numerous presently known contact lens conditioning solutions contain multiple surface-active agents. While such solutions are effective, the necessity of multiple ingredients complicates the manufacturing and regulatory process. Thus, a solution containing only one surface-active agent that provides effective conditioning would be advantageous over multiple-component systems.

For example, US Patent No. 5,773,396 to *Zhang, et al.* discloses a contact cleaning and wetting solutions containing two surfactants: one having an HLB below 18 and another having an HLB above 18. The solutions also contain an additional wetting agent, such as cellulosic materials. All of the polyoxyethylene-polyoxypropylene block copolymers disclosed therein are terminated in primary hydroxyl groups.

US Patent No. 5,209,865 to *Winterton, et al.* discloses a contact lens conditioning solution containing two surfactants: one poloxamine having an HLB of seven or below and a poloxamer also having an HLB of seven or below. All of the polyoxyethylene-polyoxypropylene block copolymers disclosed therein are terminated in primary hydroxyl groups.

Disinfecting solutions are known in the art containing only one surfactant. However, the surfactant is not employed to condition the lens, but to enhance the antimicrobial efficacy of the solution. Furthermore, none of the published references known to the applicant teach a conditioning solution containing a polyoxyethylene-polyoxypropylene block copolymer terminated in secondary hydroxyl groups.

WO 92/16244 teaches a disinfecting solution having improved antimicrobial properties containing a non-ionic surfactant, preferably polyethylene glycol fatty alcohol ethers such as PEG 24®. No mention is made of improved conditioning properties or of any polyoxyethylene-polyoxypropylene block copolymers.

WO 00/35500 teaches a disinfecting solution having improved polymeric biguanide stability. The solution contains a poloxamine surfactant having an HLB value of at least 27. No mention is made of improved conditioning properties or of any polyoxyethylene-polyoxypropylene block copolymers.

### SUMMARY OF THE INVENTION

The present invention, in one aspect, relates to a composition for conditioning a contact lens comprising an aqueous solution of:
At least one of a buffer compatible with ocular tissue or a tonicity component compatible with ocular tissue; and
a surfactant comprising a copolymer of hydrophobe and hydrophile blocks of the structure HO - (hydrophobe)ₓ, ― (hydrophile)_{y} - (hydrophobe)ₓ - H,
wherein x and y are integers reflecting the respective hydrophile and hydrophobe blocks of said copolymer;
wherein said surfactant is greater than 0.8 % by weight of the solution ; and
   wherein said surfactant is a polyoxyethylene-polyoxypropylene block copolymer terminated in secondary hydroxyl groups.

Such surfactants are generally known as "Poloxamers" and sold under the trademark "Pluronic R"® (BASF), and typically have a molecular weight of between and about 1,800 and about 9,000.

The present surfactants provide the advantages of those known in the art because the more hydrophobic portions of the polymers can adsorb more readily to the lens, and can be formulated at lower concentrations to provide better lubrication. However, they overcome the disadvantages of known surfactants because the hydrophilic portions of the polymer will not act as a substrate for deposits. Thus, the solution according to the present invention forms a uniform hydrophilic film on a lens surface for which proteins and lipids have very little affinity. As such, a contact lens contacted by the solution will have a coating that provides a prophylactic effect to the lens.

It is, therefore, an object of the present invention to provide a conditioning solution, which renders a contact lens surface more wettable so that proteins, lipids, and other tear film substituents are less likely to adhere thereto and form deposits.

It is a further object of the present invention to provide for such a solution that may be used on hard, RGP, and soft contact lenses. In another aspect, the present invention relates to a composition for conditioning a contact lens comprising an aqueous solution of:
a buffer compatible with ocular tissue;
a tonicity component; and
a surfactant comprising a copolymer of hydrophobe and hydrophile blocks of the structure: HO ― (hydrophobe)ₓ ― (hydrophile)_{y} ― (hydrophobe)ₓ ― H,
wherein x and y are integers reflecting the respective hydrophile and hydrophobe blocks of said copolymer wherein said surfactant is a polyoxyethylene-polyoxypropylene block copolymer terminated in secondary hydroxyl groups; and
wherein said composition is substantially free of a disinfecting amount of hydrogen peroxide.

Further, the present invention relates to a method for conditioning a contact lens comprising the step of contacting a contact lens with an aqueous solution comprising a polyoxyethylene-polyoxypropylene block copolymer terminated in secondary hydroxyl groups and wherein said aqueous solution is substantially free of a disinfecting amount of hydrogen peroxide.

### DETAILED DESCRIPTION OF THE PREFERRED EMBODIMENTS

The present invention provides a prophylactic action in preventing and/or retarding tear film deposits on the surfaces of contact lenses. The ingredients form a uniform hydrophilic film on the lens surface for which proteins and lipids have very little affinity. Furthermore, although some minor amounts of tear film substituents may adhere to the film, the protective film can be sacrificially removed, along with any adherence, by digitally cleaning the contact lens with any appropriate contact lens cleaner. The removal of the sacrificial film is virtually complete so that the contact lens is "renewed" to its native clean state. It is envisioned that the present solution may be used separately from other ophthalmic solutions, or may also be incorporated into a cleaning, conditioning or disinfecting solution, thus aiding in compliance with existing protocols rather than adding an extra solution, product or care step to achieve the desired prophylactic results.

The conditioning solution for contact lenses according to the present invention comprises a polyoxyethylene-polyoxypropylene nonionic surfactant terminated in secondary hydroxyl groups.

The polyoxyethylene-polyoxypropylene nonionic surfactant terminated in secondary hydroxyl groups is a surface-active agent, which will be tolerated in the formulations. Such a surfactant exhibits a high affinity for hydrophobic (lipophilic) surfaces due to the presence of the secondary hydroxyl groups on the termina of the surfactant. Thus, these surfaces active agents strongly adhere to those hydrophobic regions of the contact lens and render them hydrophilic. This adherence forms a 'barrier' to potential absorbance, and keeps them from the surface of the lens. Furthermore, this increase in hydrophilicity simultaneously decreases the thermodynamic driving force for protein and lipid absorption, thereby retarding tear film deposits.

It has been surprisingly discovered that in contrast to the teachings of the prior art, even those surfactants having a hydrophile-lipophile balance (HLB) greater than seven are suitable for use in the conditioning solution of the present invention. Preferably, the HLB of the surfactant is between 7 and 18; most preferably about 12.

The HLB of a surfactant is known to be a major factor in determining the emulsification characteristics of a non-ionic surfactant. In general, surfactants with lower HLB values are more lipophilic, while surfactants with higher HLB values are more hydrophilic. The HLB values of various polyoxyethylene-polyoxypropylene nonionic surfactants terminated in secondary hydroxyl groups are provided by BASF Performance Chemicals in published commercial literature, employed herein by reference.

Such surfactants are polyoxyethylene/ polyoxypropylene condensation polymers terminated in secondary hydroxyl groups. They may be synthesized by first creating a hydrophile (polyoxyethylene) of desired molecular weight by the controlled addition of ethylene oxide to ethylene glycol. In the second step of the synthesis, propylene oxide is added to create hydrophobic blocks on the outside of the molecule. Such block copolymers can be obtained commercially from the BASF Corporation under the trademark PLURONIC® R.

WO 02/26922, also by the present inventor, discloses disinfecting solutions containing hydrogen peroxide and polyoxyethylene-polyoxypropylene block copolymers terminated in secondary hydroxyl groups. The surfactants are taught as enhancing the antimicrobial properties of hydrogen peroxide. In contrast to the above-mentioned application, hydrogen peroxide is not a required element of the present invention; and is preferably substantially absent from the solutions of the present invention. In fact, while such agents may be present in some embodiments, the solutions of the present invention are preferably substantially free of disinfecting effective (bactericidal) amounts of antimicrobial agents such as hydrogen peroxide, quaternary ammonium agents, polymeric biguanides, benzalkonium chloride, and thimerosal. However, preferred solutions may contain non-disinfecting amounts of such agents as a preservative (bacteriostatic). While exact bactericidal and bacteriostatic amounts of the various antimicrobial agents are easily determined by one of skill in the art, by way of non-limiting example, it is noted that a preservative amount of hydrogen peroxide is typically between 10 ppm and 0.1%, while a disinfecting amount is typically greater than 1 %.

The preferred surfactant is a block copolymer of ethylene oxide and propylene oxide having the formula: 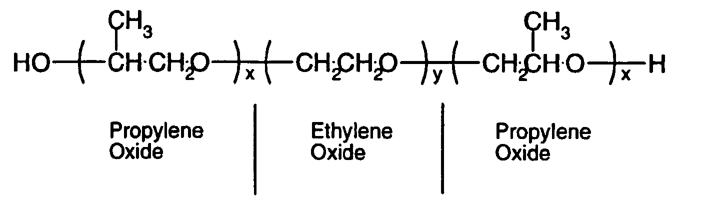 wherein x and y are integers from 1 to 350 reflecting the respective polyethylene oxide and polypropylene oxide blocks of said copolymer. The polyoxyethylene component of the block copolymer constitutes from 10 to 90 weight percent of the block copolymer. Preferably, the polyoxyethylene component of the block copolymer constitutes from 10 to 50 weight percent of the block copolymer. In another preferred embodiment of the present invention, the polyoxyethylene component of the block copolymer constitutes less than 50 weight percent of the block copolymer. Most preferably, the polyoxyethylene component of the block copolymer constitutes about 40 weight percent of the block copolymer.

Surfactants having a total molecular weight of from 1000 to 20000 are preferred. More preferred are those surfactants having a molecular weight of 1200 to 3100. Most preferred are those surfactants having a molecular weight of about 2650.

The amount of surfactant component varies over a wide range depending on a number of factors, for example, the specific surfactant or surfactants being used, the other components in the composition and the like. Often the amount is in the range of from 0.0005 % to 20 %, preferably from 0.01 % to 0.5 %. In general, the amount of surfactant is in the range of form > 0.8 % to 20 %, preferably from > 0.8 % to 10% and more preferably from > 0.8 % to 5 %.

The sequence and percent distribution of hydrophobic and hydrophilic segments in these block copolymers leads to important differences in surfactant properties. The surfactant is preferably a liquid at 20°C. The molecular weight of the polyoxypropylene block is preferably from 1200 to 3100. Preferably, molecular weight of the polyoxypropylene block is from 1000 to 2500. Most preferably, the molecular weight of the polyoxypropylene block is approximately 1700. Specific non-limiting examples of PLURONIC® R surfactants that are satisfactory include: PLURONIC® 31 R1, PLURONIC® 31 R2, PLURONIC® 25R1, PLURONIC® 17R1, PLURONIC® 17R2, PLURONIC® 12R3. Particularly good results are obtained with PLURONIC® 17R4 surfactant.

The letter 'R' found in the middle of the designation of the PLURONIC® R series signifies that this product has a reverse structure compared to the PLURONIC® products, i.e., the hydrophile (ethylene oxide) is sandwiched between the propylene oxide blocks and that the surfactant is terminated in secondary hydroxyl groups, rather than primary hydroxyl groups. The numeric designation preceding the 'R', when multiplied by 100, indicates the approximate molecular weight of the propylene oxide block. The number following the 'R', when multiplied by 10, indicates the approximate weight percent ethylene oxide in that product.

While, one of ordinary skill in the art will recognize that the surfactant of the present solution may be used in conjunction with one or more other surfactants, it is preferable to employ a polyoxyethylene-polyoxypropylene block copolymers terminated in secondary hydroxyl groups as the sole surfactant.

The conditioning solution is ocularly compatible and may be used on hard, RGP, and soft contact lenses. The active ingredients may be in any of a number of carrier vehicles. For example, the solution may be used in a soaking conditioning solution with or without a preservative or disinfecting agent.

The composition of the present invention preferably contains a buffer. The buffer maintains the pH preferably in the desired range, for example, in a physiologically acceptable range of about 4 or about 5 or about 6 to about 8 or about 9 or about 10. In particular, the solution preferably has a pH in the range of 5.5 to 8. The buffer is selected from inorganic or organic bases, preferably basic acetates, phosphates, borates, citrates, nitrates, sulfates, tartrates, lactates, carbonates, bicarbonates, tris, tris derivatives, fatty acid salts and mixtures thereof, more preferably basic phosphates, borates, citrates, tartrates, carbonates, bicarbonates and mixtures thereof. Typically, it is present in an amount of 0.001 % to 2%, preferably 0.01 % to 1%; most preferably from 0.05% to 0.30%.

The buffer component preferably includes one or more buffers, for example, combinations of monobasic phosphates, dibasic phosphates and the like. Particularly useful phosphate buffers are those selected from phosphate salts of alkali and/or alkaline earth metals. Examples of suitable phosphate buffers include one or more of sodium dibasic phosphate (Na₂HPO₄), sodium monobasic phosphate (NaH₂PO₄), and potassium monobasic phosphate (KH₂PO₄).

The present invention can be formulated in a liquid form, a near gel form, or in a gel form. One of ordinary skill would readily recognize formulations encompassed by the present disclosure suitable to achieve each form. The preferred form is a liquid form.

The solutions of the present invention preferably include an effective amount of a tonicity component to provide the liquid medium with the desired tonicity. Such tonicity components may be present in the solution and/or may be introduced into the solution. Among the suitable tonicity adjusting components that may be employed are those conventionally used in contact lens care products, such as various inorganic salts. Sodium chloride and/or potassium chloride and the like are very useful tonicity components. The amount of tonicity component included is effective to provide the desired degree of tonicity to the solution. Such amount may, for example, be in the range of from 0.4% to 1.5% (w/v). If a combination of sodium chloride and potassium chloride is employed, it is preferred that the weight ratio of sodium chloride to potassium chloride be in the range of from 3 to 6 or 8. The preferred tonicity component is sodium chloride, or its equivalent present in the range of from 0.50% to 0.90%.

Typical tonicity builders for use in the invention include suitable water soluble salts compatible with ocular tissue, preferably alkali or alkali earth metal halide, organic polyols, such as sorbitol and mannitol, sulfates, nitrates, carbonates, borates, and phosphates, more preferably sodium or potassium chloride. The tonicity builder is present in an amount sufficient to provide a tonicity of the dosage regimen of 50 to 400 mosmol/kg, most preferably 250 to 350 mosmol/kg. When non-contact lens cleaning is the desired use, the tonicity builder may also be absent or in even greater amounts than set forth above.

The lens could be soaked in the solution to allow the surfactant to absorb to the lens surface and/or the internal matrix. Alternatively, the solution may be placed in an eye drop solution, which may also be dispensed with or without a preservative or disinfecting agent. The drop would be applied to the lens while being worn on the eye. Furthermore, the conditioning solution may be placed within a contact lens cleaning solution, which would deliver the surfactant while the lens is being cleaned. In this type of formulation, the surfactant could also be used to clean, and other detergent agents would not also need to be provided, thus providing an additional advantage of that embodiment of this invention.

### Example

A quantity of the following liquid composition is prepared by blending together the individual ingredients (in g).

| | |
|---|---|
| Sodium Phosphate, Monobasic (monohydrate) | 0.772 |
| Sodium Phosphate, Dibasic (Anhydrous) | 1.555 |
| Sodium Chloride | 7.900 |
| PLURONIC® 17R4 | 0.500 |
| USP Purified Water | QS to 1 liter |

The resulting solution is an aqueous solution containing 0.0772% sodium phosphate, monobasic (monohydrate): 0.1555% sodium phosphate, dibasic (anhydrous); 0.79% sodium chloride; and 0.05% PLURONIC® 17R4. The pH is adjusted to between 6.3 and 6.6 with phosphoric acid.

## Claims

1. A composition for conditioning a contact lens comprising an aqueous solution of:
at least one of a buffer compatible with ocular tissue or a tonicity component compatible with ocular tissue; and
a surfactant comprising a copolymer of hydrophobe and hydrophile blocks of the structure:
HO ― (hydrophobe)ₓ ― (hydrophile)_{y} ― (hydrophobe)ₓ ― H
wherein x and y are integers reflecting the respective hydrophile and hydrophobe blocks of said copolymer;
wherein said surfactant is greater than 0.8% by weight of the solution; and
wherein said surfactant is a polyoxyethylene-polyoxypropylene block copolymer terminated in secondary hydroxyl groups.

2. A composition for conditioning a contact lens as claimed in Claim 2, wherein the hydrophile component of the block copolymer constitutes less than 50 weight percent of the block copolymer.

3. A composition for conditioning a contact lens as claimed in Claim 2, wherein the molecular weight of the hydrophobe block is from 1200 to 3100.

4. A composition for conditioning a contact lens as claimed in Claim 1, wherein said solution comprises a buffer selected from the group consisting of basic acetates, phosphates, borates, nitrates, sulfates, tartrates, lactates, carbonates, bicarbonates, and mixtures thereof; wherein said buffer is present in the range of 0.001% to 2%.

5. A composition for conditioning a contact lens as claimed in Claim 1, wherein said solution comprises a tonicity component providing the solution with a tonicity of from 50 to 400 mosmol/kg; wherein said tonicity component is selected from the group consisting of water soluble salts and polyols compatible with ocular tissue.

6. A composition for conditioning a contact lens as claimed in Claim 1, wherein said surfactant has an HLB greater than 7.

7. A composition for conditioning a contact lens comprising an aqueous solution of:
a buffer compatible with ocular tissue;
a tonicity component; and
a surfactant comprising a copolymer of hydrophobe and hydrophile blocks of the structure:
HO ― (hydrophobe)ₓ― (hydrophile)_{y}― (hydrophobe)ₓ - H,
wherein x and y are integers reflecting the respective hydrophile and hydrophobe blocks of said copolymer,
wherein said surfactant is a polyoxyethylene-polyoxypropylene block copolymer terminated in secondary hydroxyl groups; and
wherein said composition is substantially free of a disinfecting amount of hydrogen peroxide.

8. A composition for conditioning a contact lens as claimed in Claim 7, wherein said composition further comprises hydrogen peroxide in an amount between 10 ppm and 0.1%.

9. A composition for conditioning a contact lens as claimed in Claim 8, wherein the hydrophile constitutes about 40 weight percent of the block copolymer.

10. A composition for conditioning a contact lens as claimed in Claim 7, wherein the molecular weight of the hydrophobe is approximately 1700.

11. A composition for conditioning a contact lens as claimed in Claim 4, wherein said phosphate buffer is selected from the group consisting of monobasic phosphates, dibasic phosphates, and mixtures thereof; wherein said phosphate buffer is present in the range of from 0.005% to 0.30%.

12. A composition for conditioning a contact lens as claimed in Claim 7, wherein said tonicity component provides the solution with a tonicity of from 50 to 400 mosmol/kg; wherein said tonicity component is selected from the group consisting of water soluble salts compatible with ocular tissue.

13. A composition for conditioning a contact lens as claimed in Claim 7, wherein said composition is substantially free of surface active agents other than said surfactant.

14. A method for conditioning a contact lens comprising the step of contacting a contact lens with an aqueous solution comprising a polyoxyethylene-polyoxypropylene block copolymer terminated in secondary hydroxyl groups and wherein said aqueous solution is substantially free of a disinfecting amount of hydrogen peroxide,

15. A method for conditioning a contact lens as claimed in Claim 14, wherein said aqueous solution is substantially free of surface active agents other than said polyoxyethylene-polyoxypropylene block copolymer.

16. A method for conditioning a contact lens as claimed in Claim 14, wherein said polyoxyethylene-polyoxypropylene block copolymer has an HLB greater than 7.

## Patentansprüche

1. Zusammensetzung zum Konditionieren einer Kontaktlinse, umfassend eine wässrige Lösung von:
mindestens einen von einem Puffer, der mit Okulargewebe verträglich ist, oder einer Tonizitätskomponente, die mit Okulargewebe verträglich ist; und
ein Tensid, umfassend ein Copolymer von hydrophoben und hydrophilen Blöcken der Struktur:
HO― (hydrophob)ₓ - (hydrophil)_{y} - (hydrophob)ₓ - H,
worin x und y ganze Zahlen sind, die die jeweiligen hydrophilen und hydrophoben Blöcke des Copolymers wiedergeben;
wobei das Tensid größer als 0,8 Gewichtsprozent der Lösung ist; und
worin das Tensid ein Polyoxyethylen-Polyoxypropylen-Blockcopolymer darstellt, das in sekundären Hydroxylgruppen beendet ist.

2. Zusammensetzung zum Konditionieren einer Kontaktlinse nach Anspruch 2, wobei die hydrophile Komponente des Blockcopolymers weniger als 50 Gewichtsprozent des Blockcopolymers ausmacht.

3. Zusammensetzung zum Konditionieren einer Kontaktlinse nach Anspruch 2, wobei das Molekulargewicht des hydrophoben Blocks 1200 bis 3100 ist.

4. Zusammensetzung zum Konditionieren einer Kontaktlinse nach Anspruch 1, wobei die Lösung einen Puffer, ausgewählt aus der Gruppe, bestehend aus basischen Acetaten, Phosphaten, Boraten, Nitraten, Sulfaten, Tartraten, Lactaten, Carbonaten, Bicarbonaten ,und Gemischen davon umfasst, wobei der Puffer im Bereich von 0,001 % bis 2 % vorliegt.

5. Zusammensetzung zum Konditionieren einer Kontaktlinse nach Anspruch 1, wobei die Lösung eine Tonizitätskomponente umfasst, die die Lösung mit einer Tonizität von 50 bis 400 mOsmol/kg versieht; wobei die Tonizitätskomponente aus der Gruppe, bestehend aus in Wasser löslichen Salzen und Polyolen, die mit Okulargewebe verträglich sind, ausgewählt ist.

6. Zusammensetzung zum Konditionieren einer Kontaktlinse nach Anspruch 1, wobei das Tensid einen HLB-Wert größer als 7 aufweist.

7. Zusammensetzung zum Konditionieren einer Kontaktlinse, umfassend eine wässrige Lösung von:
einem Puffer, der mit Okulargewebe verträglich ist;
einer Tonizitätskomponente; und
einem Tensid, umfassend ein Copolymer von hydrophoben und hydrophilen Blöcken der Struktur:
HO― (hydrophob)ₓ ― (hydrophil)_{y} ― (hydrophob)ₓ ― H,
worin x und y ganze Zahlen sind, die die jeweiligen hydrophilen und hydrophoben Blöcke des Copolymers wiedergeben;
wobei das Tensid ein Polyoxyethylen-Polyoxypropylen-Blockcopolymer darstellt, das in sekundären Hydroxylgruppen beendet ist; und
wobei die Zusammensetzung im Wesentlichen frei von einer desinfizierenden Menge an Wasserstoffperoxid ist.

8. Zusammensetzung zum Konditionieren einer Kontaktlinse nach Anspruch 7, wobei die Zusammensetzung weiterhin Wasserstoffperoxid in einer Menge zwischen 10 ppm und 0,1 % umfasst.

9. Zusammensetzung zum Konditionieren einer Kontaktlinse nach Anspruch 8, wobei das Hydrophile etwa 40 Gewichtsprozent des Blockcopolymers ausmacht.

10. Zusammensetzung zum Konditionieren einer Kontaktlinse nach Anspruch 7, wobei das Molekulargewicht des Hydrophoben ungefähr 1700 ist.

11. Zusammensetzung zum Konditionieren einer Kontaktlinse nach Anspruch 4, wobei der Phosphatpuffer aus der Gruppe, bestehend aus einbasigen Phosphaten, zweibasigen Phosphaten und Gemischen davon, ausgewählt ist, wobei der Phosphatpuffer im Bereich von 0,005 % bis 0,30 % vorliegt.

12. Zusammensetzung zum Konditionieren einer Kontaktlinse nach Anspruch 7, wobei die Tonizitätskomponente die Lösung mit einer Tonizität von 50 bis 400 mOsmol/kg versieht;
wobei die Tonizitätskomponente aus der Gruppe, bestehend aus in Wasser löslichen Salzen, die mit Okulargewebe verträglich sind, ausgewählt ist.

13. Zusammensetzung zum Konditionieren einer Kontaktlinse nach Anspruch 7, wobei die Zusammensetzung im Wesentlichen frei von oberflächenaktiven Mitteln ist, die von dem Tensid verschieden sind.

14. Verfahren zum Konditionieren einer Kontaktlinse, umfassend den Schritt des In-Kontakt-Bringens einer Kontaktlinse mit einer wässrigen Lösung, umfassend ein Polyoxyethylen-Polyoxypropylen-Blockcopolymer, das in sekundären Hydroxylgruppen beendet ist, und wobei die wässrige Lösung im Wesentlichen frei von einer desinfizierenden Menge an Wasserstoffperoxid ist.

15. Verfahren zum Konditionieren einer Kontaktlinse nach Anspruch 14, wobei die wässrige Lösung im Wesentlichen frei von oberflächenaktiven Mitteln ist, die von dem Polyoxyethylen-Polyoxypropylen-Blockcopolymer verschieden sind.

16. Verfahren zum Konditionieren einer Kontaktlinse nach Anspruch 14, wobei das Polyoxyethylen-Polyoxypropylen-Blockcopolymer einen HLB-Wert von größer als 7 aufweist.

## Revendications

1. Composition pour conditionner une lentille de contact, comprenant une solution aqueuse
d'au moins l'un d'un tampon compatible avec le tissu oculaire, ou d'un composant de tonicité compatible avec le tissu oculaire ; et
d'un tensioactif comprenant un copolymère à blocs hydrophobes et hydrophiles ayant la structure HO-(hydrophobe)ₓ-(hydrophile)_{y}-(hydrophobe)ₓ-H
dans laquelle x et y sont des entiers qui correspondent respectivement aux blocs hydrophiles et hydrophobes dudit copolymère ;
où la quantité dudit tensioactif est supérieure à 0,8 % en poids par rapport à la solution ; et
où ledit tensioactif est un copolymère à blocs polyoxyéthylène-polyoxypropylène, terminé par des groupes hydroxyle secondaires.

2. Composition pour conditionner une lentille de contact selon la revendication 2, dans laquelle le composant hydrophile du copolymère à blocs représente moins de 50 % en poids du copolymère à blocs.

3. Composition pour conditionner une lentille de contact selon la revendication 2, dans laquelle la masse moléculaire du bloc hydrophobe est de 1200 à 3100.

4. Composition pour conditionner une lentille de contact selon la revendication 1, dans laquelle ladite solution comprend un tampon choisi dans l'ensemble consistant en les acétates, phosphates, borates, nitrates, sulfates, tartrates, lactates, carbonates, bicarbonates basiques et leurs mélanges ; où ledit tampon est présent en une quantité comprise dans la plage de 0,001 à 2 %.

5. Composition pour conditionner une lentille de contact selon la revendication 1, dans laquelle ladite solution comprend un composant de tonicité conférant à la solution une tonicité de 50 à 400 mosmoles/kg ; où ledit composant de tonicité est choisi dans l'ensemble consistant en les sels solubles dans l'eau et les polyols compatibles avec le tissu oculaire.

6. Composition pour conditionner une lentille de contact selon la revendication 1, dans laquelle ledit tensioactif a un rapport HLB supérieur à 7.

7. Composition pour conditionner une lentille de contact, comprenant une solution aqueuse :
d'un tampon compatible avec le tissu oculaire ;
d'un composant de tonicité, et
d'un tensioactif comprenant un copolymère à blocs hydrophobes et hydrophiles ayant la structure HO-(hydrophobe)ₓ-(hydrophile)_{y}-(hydrophobe)ₓ-H,
où x et y sont des entiers correspondant respectivement aux blocs hydrophiles et hydrophobes dudit copolymère,
où ledit tensioactif est un copolymère à blocs polyoxyéthylène-polyoxypropylène terminé par des groupes hydroxyle secondaires ; et
où ladite composition est pour ainsi dire exempte d'une quantité désinfectante de peroxyde d'hydrogène.

8. Composition pour conditionner une lentille de contact selon la revendication 7, dans laquelle ladite composition comprend en outre du peroxyde d'hydrogène en une quantité comprise entre 10 ppm et 0,1 %.

9. Composition pour conditionner une lentille de contact selon la revendication 8, dans laquelle les blocs hydrophiles constituent environ 40 % en poids du copolymère à bloc.

10. Composition pour conditionner une lentille de contact selon la revendication 7, dans laquelle la masse moléculaire du bloc hydrophobe est d'environ 1700.

11. Composition pour conditionner une lentille de contact selon la revendication 4, dans laquelle ledit tampon phosphate est choisi dans l'ensemble consistant en les phosphates monobasiques, les phosphates dibasiques et leurs mélangés ; et où ledit tampon phosphate est présent en une quantité comprise dans la plage de 0,005 à 0,30 %.

12. Composition pour conditionner une lentille de contact selon la revendication 7, dans laquelle ledit composant de tonicité confère à la solution une tonicité de 50 à 400 mosmoles/kg ; où ledit composant de tonicité est choisi dans l'ensemble consistant en les sels solubles dans l'eau compatibles avec le tissu oculaire.

13. Composition pour conditionner une lentille de contact selon la revendication 7, dans laquelle ladite composition est pour ainsi dire exempte d'agents tensioactifs autres que ledit tensioactif.

14. Procédé pour conditionner une lentille de contact, comprenant l'étape consistant à mettre en contact une lentille de contact avec une solution aqueuse contenant un copolymère à blocs polyoxyéthylène-polyoxypropylène terminé par des groupes hydroxyle secondaires, et où ladite solution aqueuse est pour ainsi dire exempte d'une quantité désinfectante de peroxyde d'hydrogène.

15. Procédé pour conditionner une lentille de contact selon la revendication 14, dans laquelle ladite solution aqueuse est pour ainsi dire exempte d'agents tensioactifs autres que ledit copolymère à blocs polyoxyéthylène-polyoxypropylène.

16. Procédé pour conditionner une lentille de contact selon la revendication 14, dans laquelle ledit copolymère à blocs polyoxyéthylène-polyoxypropylène a un rapport HLB supérieur à 7.
